# EUROPEAN PATENT SPECIFICATION

(11) **EP 1 115 684 B1**
(45) Date of publication and mention of the grant of the patent: **18.12.2002**
(21) Application number: 99969406.0
(22) Date of filing: 15.09.1999
(51) Int. Cl.: C07C 51/10, C07C 53/128

(54) **PROCESS FOR THE MANUFACTURE OF QUATERNARY CARBOXYLIC ACIDS**
VERFAHREN ZUR HERSTELLUNG VON QUARTÄREN CARBONSÄUREN
PROCEDE SERVANT A PREPARER DES ACIDES CARBOXYLIQUES QUATERNAIRES

(30) Priority: 21.09.1998 EP 98203165
(43) Date of publication of application: 18.07.2001
(73) Proprietor: Resolution Research Nederland B.V., 1031 CM Amsterdam (NL)
(72) Inventor: LANGE, Jean-Paul, NL-1031 CM Amsterdam (NL); OTTEN, Vincent, NL-1031 CM Amsterdam (NL)
(74) Representative: van der Straaten, Jan Anthony
(86) International application number: EP9906998
(87) International publication number: WO00017146

(56) References cited:
- WO-A-96/20154
- WO-A-98/38149

## Description

The invention relates to a process for the manufacture of quaternary carboxylic acids. More in particular the invention relates to a process for the manufacture of quaternary carboxylic acids from higher linear olefins by means of a Koch synthesis using carbon monoxide as reagent and a solid acid catalyst.

It is known from e.g. New Synthesis with Carbon Monoxide, J. Falbe, Springer Verlag, Berlin, Reactivity and Structure, Concepts in Organic Chemistry 11, 1980, p. 276, 376 and 377, to produce branched carboxylic acids by hydrocarboxylation of linear olefins using homogeneous catalyst systems.

However such homogeneously catalyzed hydrocarboxylation reactions had as important disadvantages that mixtures of many carboxylic acid components in varying proportions were obtained and that a separation step to isolate and recover the expensive catalyst system was always necessary.

It will be appreciated that there has developed a strong need for an efficient manufacturing process for quaternary carboxylic acids, starting from linear olefins and using a solid catalyst system.

The up to now available processes are characterized by the fact that no solid acid catalyst could be used, unless said catalyst is operated under unattractively severe conditions or unless said catalyst is combined with corrosive Lewis acid cocatalyst or unless said catalyst is used in a non-aqueous reaction system.

In particular from International Application WO 96/20154 was known a process for the production of trialkylacetic acids from branched olefins and carbon monoxide in a non-aqueous reaction system using a solid resin catalyst comprising a cationic resin, having sufficient acid groups to provide requisite protons for conversion of branched olefin and carbon monoxide to trialkylacetic acids.

In particular the cationic resin was specified to have an acidity of at least equivalent to that of a 65 wt% sulphuric acid.

It will be appreciated by an average person skilled in the art that said process can only be performed in two steps, i.e. one step comprising contacting the solid catalyst with olefin/CO feed and a subsequent step contacting the catalyst with water feed, and that stoichiometric amounts of branched olefin and water will not lead to the desired products in an acceptable yield. Moreover, said process cannot produce more than 1 mole of converted olefin per mole active proton on the solid catalyst in one cycle of two steps.

On the other hand from WO 92/18592 was known a process for the manufacture of trialkylacetic acids and particularly of pivalic acid, from branched olefins and particularly isobutene, and Carbon monoxide, using a solid acid catalyst together with minor amounts of a Lewis acid, such as boron trifluoride.

In addition, from EP-A-0249976 was known a process for the manufacture of branched carboxylic acids, by catalytic conversion of olefins with carbon monoxide and water in the presence of zeolites as catalysts at temperatures of from 200 to 500 °C and at pressures of 200 to 700 bar.

More in particular zeolites of the pentasil type are used as catalysts. According to the exemplified embodiments only high temperatures (300 °C) and pressures (300-500 bar) are used.

It will be appreciated that said disclosed reaction conditions will give rise to higher operation costs due to required measures as to safety and environment.

WO 98/38149 actually disclosed a process for the manufacture of branched carboxylic acids from branched olefins by reaction with carbon monoxide (pressure 50 to 100 bar) and water (in a molar ratio water/olefin in the range of from 0.5 to 2 mole/mole), in the presence of a solid acidic ion exchange resin, such as sulfonated resins and sulfonated siloxane polymers, having an acid strength equivalent per active site of at least 65 wt%, and in the presence of a polar non-coordinating organic solvent and more preferably a carboxylic acid as solvent. Not any suggestion occurred for the conversion of unbranched olefins.

An object of the present invention is providing an alternative efficient one step manufacturing process for quaternary carboxylic acids, which process starts from linear olefins containing 4 or more carbon atoms, and which uses a solid catalyst system under relatively mild conditions on the one hand and which shows economically acceptable conversion and economically acceptable selectivity to quaternary carboxylic acids on the other hand.

As a result of extensive research and experimentation there has now been surprisingly found a one step process for manufacture of quaternary carboxylic acids from linear olefins by means of reaction with carbon monoxide and a solid sulfonic acidic ion exchanger resin having an acid strength equivalent to at least 65 wt% sulphuric acid, characterized in that a linear olefin containing 4 or more carbon atoms, or a precursor thereof, is reacted in a batch reactor or a continuous reactor with carbon monoxide and water, in the presence of a solid sulfonic acidic ion exchanger resin having an acid strength equivalent to at least 65 wt% sulphuric acid and carbon monoxide into quaternary carboxylic acids, and in the presence of a polar non-coordinating organic solvent.

More in particular the invention relates to an improved manufacturing process of trialkylacetic acids of the formula 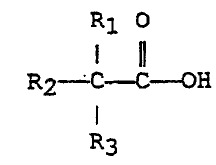 wherein each symbol R represents a radical having 1 to 10 carbon atoms.

Preferably the total number of carbon atoms in the trialkylacetic acids ranges from 5 to 19 and more preferably from 5 to 14 carbon atoms and most preferably from 9 to 13.

With the term "linear olefin or a precursor thereof" as used throughout the present specification is meant that the specified linear olefin itself as well as alcohols, esters or ethers, from which the specific olefin can be easily derived, can be used as starting materials for the present manufacturing process, which makes this process much more flexible than conventional prior art processes.

More in particular linear olefins, containing from 4 to 15 carbon atoms and more preferably from 8 to 10 carbon atoms or precursors therefor, can be converted by the present process into quaternary carboxylic acids aimed at.

An important advantage of the present process is that it can be operated as one step or one reactor process showing an economically acceptable combination of conversion degree and selectivity.

The catalyst to be used for the process of the present invention is a solid sulfonic acidic ion exchanger resin having an acid strength equivalent to at least 65 wt% sulphuric acid. It is preferably selected from the group consisting of sulfonated copolymers of styrene and divinylbenzene, copolymers of vinylnaphthalene and divinylbenzene, copolymers of styrene and methacrylic acid resins, phenolic based resins, sulfonated poly(tetrafluoroethylene) and sulfonated siloxane polymers and sulfonated cellulose derivatives.

The solid acidic ion exchange resin is treated to give a sulfonic acid cation-exchange resin capable of providing sufficient protons, i.e. the resin having an acid strength equivalent to at least 65 wt% sulphuric acid and preferably to at least 70 wt% sulphuric acid.

Catalyst solid resins, comprising sulfonic acid groups and derived from copolymers from styrene-divinylbenzene, copolymers from vinylnaphthalene-divinyl benzene or derived from (tetrafluoroethylene)polymers or from siloxane polymers are preferred.

Specific more preferred examples of commercial effective acidic catalysts are AMBERLYST, NAFION or DELOXAN catalysts (AMBERLYST, NAFION and DELOXAN are Trade Marks).

Most preferred are styrene-divinylbenzene copolymer based catalyst such as the AMBERLYST type catalysts. More preferably AMBERLYST 38 catalyst is used. The reaction temperature in the batch reactor is in the range of from 25 °C to 200 °C and preferably from 100 to 150 °C.

The pressure in the reactor is in the range of from 1 to 200 bar and preferably from 50 to 100 bar.

As polar non-coordinating organic solvents can be used chemically inert polar organic solvents such as carboxylic acids or derivatives thereof and more in particular esters, or an optionally substituted sulfolane (preferably sulfolane).

According to a more preferred embodiment of the present process, as polar non-coordinating solvent a quaternary acid is present in the continuous reactor and preferably a CSTR reactor. Most preferably the carboxylic acid to be produced can be used as solvent.

Normally the CSTR reactor is filled with solvent and catalyst with a catalyst/solvent wt ratio of in the range of from 0.01 to 0.5 w/w solid/liquid and preferably 0.2-0.3 w/w. The other respective reactants are introduced into the reactor and reaction mixture is heated to the desired 5-30 mmol/reaction temperature.

Alternatively for a fixed bed reactor with liquid recycling can be operated with a catalyst/solvent ratio up to 0.95 w/w (solid/liquid) and preferably in the range of from 0.4 to 0.8.

The feed of starting olefin is in the range of from 15 g cat. 0.3 to 2 mmol/g catalyst and preferably from 0.6 to 1.5 mmol/g catalyst, while the water/olefin molar ratio or the molar ratio of the respective precursors therefor is in the range of from 0.5 to 2 mole/mole and preferably 1 and the CO/olefin molar ratio is in the range of from 0.5 to 1000 mole/mole and preferably from 1 to 100.

It will be appreciated that, when using water amounts significantly below the hereinbefore specified amounts, the process becomes unattractive due to too low selectivity and that the selectivity and conversion have surprisingly been improved when using stoichiometric water:olefin = 1:1 feed.

The invention is further illustrated by the following examples, however without restricting its scope to these specific embodiments.

### Example 1: 2-octanol in VERSATIC 11 acids

AMBERLYST 38 was dried overnight in an oven at 100 °C and a sample of 15.3 g dry AMBERLYST was loaded in a 250 ml autoclave together with 49.9 g branched C₁₁ acids (VERSATIC 11) as solvent and 80 bar CO. The autoclave was heated up to 150 °C and kept under constant pressure by means of the use of a constant back-pressure regulator and a constant gas flow of 1.35 l.CO/h. 2-octanol was continuously fed to the autoclave at a rate of 2.29 ml/h (14.13 mmol/h) during 22.0 h. The autoclave was then cooled to room temperature, depressurized and unloaded.

A sample of the product mixture was analyzed by means of gas chromatography. The carboxylic acids were extracted from the remaining fraction by means of washing with an equivalent volume of 4M NaOH solution, acidification of the NaOH extract to pH=1 with HCl, extraction of the carboxylic acids with an equivalent volume of diethyl ether and evaporation of the ether under mild heating. The concentrated carboxylic acid mixture was analyzed by means of gas chromatography.

The total product mixture contained 14 C% carboxylic acids other than branched C₁₁ acids (VERSATIC 11 acids) used as solvent. This corresponds to a yield in non-C₁₁ carboxylic acid of 46 C%, based on feed. The extracted fraction contained 94 C% of branched C₉ acids (VERSATIC 9 acids), after renormalization to exclude the branched C₁₁ acids (VERSATIC 11 acids) used as solvent.

### Example 2: 2-pentanol in VERSATIC 11 acids

AMBERLYST 38 was dried overnight in an oven at 100 °C and a sample of 15.2 g dry AMBERLYST was loaded in a 250 ml autoclave together with 51.7 g branched C₁₁ acids (VERSATIC 11) as solvent and 80 bar CO. The autoclave was heated up to 150 °C and kept under constant pressure by means of the use of a constant back-pressure regulator and a constant gas flow of 1.35 l.CO/h. 2-pentanol was continuously fed to the autoclave at a rate of 1.61 ml/h (14.9 mmol/h) during 19.0 h. The autoclave was then cooled to room temperature, depressurized and unloaded. The reaction product was analyzed as described in example 1.

The total product mixture contained 14 C% carboxylic acids other than branched C₁₁ acids (VERSATIC 11 acids) used as solvent. This corresponds to a yield in non-C₁₁ carboxylic acid of 71 C%, based on feed. The extracted fraction contained 85 C% of branched C₆ acids (VERSATIC 6), after renormalization to exclude the branched C₁₁ acids (VERSATIC 11 acids) used as solvent.

### Example 3: 2-pentanol in VERSATIC 5 acids

AMBERLYST 38 was dried overnight in an oven at 100 °C and a sample of 16.5 g dry AMBERLYST was loaded in a 250 ml autoclave together with 57.4 g pivalic acid as solvent and 80 bar CO. The autoclave was heated up to 150 °C and kept under constant pressure by means of the use of a constant back-pressure regulator and a constant gas flow of 1.35 l.CO/h. 2-pentanol was continuously fed to the autoclave at a rate of 1.63 ml/h (15.1 mmol/h) during 21.0 h. The autoclave was then cooled to room temperature, depressurized and unloaded. The reaction product was analyzed as described in example 1.

The total product mixture contained 14 C% carboxylic acids other than pivalic acid used as solvent. This corresponds to a yield in carboxylic acid (other than pivalic acid) of 54 C%, based on feed. The extracted fraction contained 82 C% of branched C₆ acids (VERSATIC 6 acids), after renormalization to exclude the pivalic acid used as solvent.

### Comparative Example 1: DIBC in VERSATIC 11 acids

AMBERLYST 38 was dried overnight in an oven at 100 °C and a sample of 15.4 g dry AMBERLYST was loaded in a 250 ml autoclave together with 54.1 g branched C₁₁ acids (VERSATIC acid 11) as solvent and 80 bar CO. The autoclave was heated up to 150 °C and kept under constant pressure by means of the use of a constant back-pressure regulator and a constant gas flow of 1.35 l.CO/h. Diisobutyl-carbinol (DIBC) was continuously fed to the autoclave at a rate of 2.25 ml/h (12.6 mmol/h) during 17.0 h. The autoclave was then cooled to room temperature, depressurized and unloaded. The reaction product was analyzed as described in example 1.

The total product mixture contained 15 C% carboxylic acids other than branched C₁₁ acids (VERSATIC 11 acids) used as solvent. This corresponds to a yield in non-C₁₁ carboxylic acid of 64 C%, based on feed. The extracted fraction contained 95 C% of branched C₁₀ acids (VERSATIC 10 acids), after renormalization to exclude the branched C₁₁ acids (VERSATIC 11 acids) used as solvent.

## Claims

1. A process for manufacture of quaternary carboxylic acids from linear olefins, by means of reaction with carbon monoxide and a solid acid catalyst, **characterized in that** a linear olefin, containing 4 or more carbon atoms or a precursor thereof, is reacted in a batch reactor or continuous reactor with carbon monoxide and water, in the presence of a solid sulfonic acidic ion exchanger resin having an acid strength equivalent to at least 65 wt% sulphuric acid, and carbon monoxide into quaternary carboxylic acids, and in the presence of a polar non-coordinating organic solvent.

2. A process according to claim 1, **characterized in that** as solid acid catalyst is used a solid acidic ion exchanger, selected from the group consisting of sulfonated copolymers from vinylnaphthalene-divinylbenzene or styrene-divinyl benzene, sulfonated poly(tetrafluoro-ethylene) resins and sulfonated siloxane polymers.

3. A process according to claim 1, **characterized in that** the resin is treated to give a sulfonic acid cation-exchange resin, such that the resin having an acid strength equivalent to at least 70 wt% sulphuric acid.

4. A process according to claims 1-3, **characterized in that** the pressure in the reactor is in the range of from 50 to 100 bar.

5. A process according to claims 1-4, **characterized in that** during the reaction a carboxylic acid or a derivative thereof is present as solvent in the reactor.

6. A process according to claims 1-5, **characterized in that** the catalyst/solvent weight ratio is in the range of from 0.01 to 0.5 w/w for a CSTR reactor.

7. A process according to claims 1-5, **characterized in that** the catalyst/solvent weight ratio is in the range of from 0.4 to 0,8 w/w for a fixed reactor with liquid recycling.

8. A process according to claims 1-7, **characterized in that** the water/olefin molar ratio or the molar ratio of the respective precursor therefor is in the range of from 0.5 to 2 mole/mole.

9. A process according to claims 1-8, **characterized in that** the CO/olefin molar ratio is in the range of from 0.5 to 1000 mole/mole.

## Patentansprüche

1. Verfahren zum Herstellen von quatemären Carbonsäuren aus linearen Olefinen mittels Reaktion mit Kohlenmonoxid und einem festen Säurekatalysator, **dadurch gekennzeichnet, daß** ein lineares Olefin, das mindestens 4 Kohlenstoffatome oder eine Vorstufe davon aufweist, in einem diskontinuierlichen Reaktor oder einem kontinuierlichen Reaktor mit Kohlenmonoxid und Wasser in Gegenwart eines festen Sulfonsäure-Ionenaustauschharzes, das ein Säurestärkeäquivalent von mindestens 65 Masseprozent Schwefelsäure aufweist, und in Gegenwart eines sich polar nicht koordinativ anlagernden organischen Lösungsmittels, zu quaternären Carbonsäuren reagiert.

2. Ein Verfahren gemäß Anspruch 1, **dadurch gekennzeichnet, daß** ein fester Säure-Ionenaustauscher als fester Säurekatalysator verwendet wird, wobei der feste Säure-Ionenaustauscher aus der Gruppe ausgewählt wird, die aus sulfonierten Copolymeren von Vinyl-Naphtalen-Divinylbenzolen oder Styren-Divinylbenzolen, sulfonierten Poly(tetrafluorethen)harzen und sulfonierten Siloxanpolymeren bestehen.

3. Ein Verfahren gemäß Anspruch 1, **dadurch gekennzeichnet, daß** das Harz behandelt wird, um ein Sulfonsäure-Kationenaustauschharz derart zu ergeben, daß das Harz ein Säurestärkeäquivalent von mindestens 70 Masseprozent Schwefelsäure aufweist.

4. Ein Verfahren gemäß den Ansprüchen 1 - 3, **dadurch gekennzeichnet, daß** der Druck in dem Reaktor in einem Bereich von 50 bis 100 bar liegt.

5. Ein Verfahren gemäß den Ansprüchen 1 - 4, **dadurch gekennzeichnet, daß** während der Reaktion eine Carbonsäure oder eines ihrer Derivate als Lösungsmittel in dem Reaktor vorhanden ist.

6. Ein Verfahren gemäß den Ansprüchen 1 - 5, **dadurch gekennzeichnet, daß** für einen CSTR-Reaktor das Katalysator/Lösungsmittel-Masseverhältnis in einem Bereich von 0,01 bis 0,5 Masse/Masse liegt.

7. Ein Verfahren gemäß den Ansprüchen 1 - 5, **dadurch gekennzeichnet, daß** für einen Festbettreaktor mit Flüssigkreislaufführung das Katalysator/Lösungsmittel-Masseverhältnis in einem Bereich von 0,4 bis 0,8 Masse/Masse liegt.

8. Ein Verfahren gemäß den Ansprüchen 1 - 7, **dadurch gekennzeichnet, daß** das Wasser/Olefin-Molverhältnis oder das Molverhältnis der diesen entsprechenden Vorstufen in einem Bereich von 0,5 bis 2 Mol/Mol liegt.

9. Ein Verfahren gemäß den Ansprüchen 1 - 8, **dadurch gekennzeichnet, daß** das CO/Olefin-Molverhältnis in einem Bereich von 0,5 bis 1000 Mol/Mol liegt.

## Revendications

1. Un procédé pour la fabrication d'acides carboxyliques quaternaires à partir d'oléfines linéaires, en utilisant la réaction avec le monoxyde de carbone et un catalyseur acide solide, **caractérisé par le fait que** l'oléfine linéaire, comprenant 4 atomes de carbone ou davantage ou un précurseur, soit placée pour réaction dans un réacteur discontinu, ou dans un réacteur continu avec du monoxyde de carbone et de l'eau, en présence d'une résine solide échangeuse d'ions d'acide sulfonique, d'une force acide au moins équivalente de l'acide sulfurique 65 wt%, dans les acides carboxyliques quaternaires, et en présence d'un solvant organique non coordonné polaire.

2. Un procédé selon la revendication 1, **caractérisé par le fait qu'**un échangeur d'ions acide solide est utilisé comme catalyseur acide solide, sélectionné dans le groupe composé de copolymères sulfonés de vinylnaphtalène-divinylbenzène ou de styrène-divinyl benzène, de résines poly (tétrafluoroéthylène) sulfonés et de polymères de siloxane sulfonés.

3. Un procédé selon la revendication 1, **caractérisé par le fait que** la résine est traitée pour donner une résine échangeuse de cations d'acide sulfonique, de façon telle que la résine présente une force acide au moins équivalente à l'acide sulfurique 70 wt%.

4. Un procédé selon les revendications 1 à 3, **caractérisé par le fait que** la pression dans le réacteur est de l'ordre de 50 à 100 bar.

5. Un procédé selon les revendications 1 à 4, **caractérisé par le fait que** pendant la réaction, un acide carboxylique ou un de ses dérivés est présent en tant que solvant, dans le réacteur.

6. Un procédé selon les revendications 1 à 5, **caractérisé par le fait que** le rapport de poids catalyseur/solvant est de l'ordre de 0,01 à 0,5 poids/poids pour un réacteur CSTR.

7. Un procédé selon les revendications 1 à 5, **caractérisé par le fait que** le rapport de poids catalyseur/solvant est de l'ordre de 0,4 à 0,8 poids/poids pour un réacteur fixe avec recyclage du

8. Un procédé selon les revendications 1 à 7, **caractérisé par le fait que** le rapport molaire eau/oléfine ou le rapport molaire de leurs précurseurs respectifs est de l'ordre de 0,5 à 2 mole/mole.

9. Un procédé selon les revendications 1 à 8, **caractérisé par le fait que** le rapport molaire CO/oléfine est de l'ordre de 0,5 à 1000 mole/mole.
